# EUROPÄISCHE PATENTSCHRIFT

(11) **EP 1 154 794 B1**
(45) Veröffentlichungstag und Bekanntmachung des Hinweises auf die Patenterteilung: **23.04.2003**
(21) Anmeldenummer: 98964385.3
(22) Anmeldetag: 09.12.1998
(51) Int. Cl.: A61K 45/06, A61K 31/195, A61K 31/445, A61K 31/245, A61P 25/16

(54) **BEHANDLUNG DER SYMPTOME DER PARKINSONSCHEN KRANKHEIT MIT EINEM MITTEL ENTHALTEND EINE DOPAMINERGE SUBSTANZ UND EIN LOKALANAESTHETIKUM DER ANILID-GRUPPE**
TREATMENT OF SYMPTOMS OF PARKINSON'S DISEASE WITH AN AGENT COMPRISING A DOPAMINERGIC SUBSTANCE AND A LOCAL ANAESTHETIC OF THE ANILIDE GROUP
TRAITEMENT DES SYMPTOMES DE LA MALADIE DE PARKINSON AVEC UN AGENT CONTENANT UNE SUBSTANCE DOPAMINERGIQUE ET UN ANESTHESIQUE LOCAL DU GROUPE ANILIDE

(30) Priorität: 03.12.1998 DE 19855704
(43) Veröffentlichungstag der Anmeldung: 21.11.2001
(73) Patentinhaber: Saiger, Lothar, 88525 Dürmentingen (DE)
(72) Erfinder: Saiger, Lothar, 88525 Dürmentingen (DE)
(74) Vertreter: Holzmüller, Reinhold, Dr.
(86) Internationale Anmeldenummer: DE9803612
(87) Internationale Veröffentlichungsnummer: WO00032232

(56) Entgegenhaltungen:
- EP-A- 0 878 191
- WO-A-96/41616
- WO-A-98/00142
- US-A- 5 190 763
- US-A- 5 484 608
- US-A- 5 668 117
- MURA A ET AL: "Reevaluation of the Striatal Role in the Expression of Turning Behavior in the Rat Model of Parkinson 's Disease." BRAIN RESEARCH, (1998 OCT 12) 808 (1) 48-55. , XP002116997
- BATHIEN N ET AL: "Reciprocal continuous inhibition in rigidity of Parkinsonism." JOURNAL OF NEUROLOGY, NEUROSURGERY AND PSYCHIATRY, (1977 JAN) 40 (1) 20-4 , XP002116998
- FURUYA R ET AL: "Successful perioperative management of a patient with Parkinson 's disease by enteral levodopa administration under propofol anesthesia." ANESTHESIOLOGY, (1998 JUL) 89 (1) 261-3. , XP002116999
- SALATA J J ET AL: "Amantadine-induced diastolic depolarization and automaticity in ventricular muscle." CIRCULATION RESEARCH, (1982 DEC) 51 (6) 722-32. , XP002117000
- DOSTROVSKY J O ET AL: "Microinjection of lidocaine into human thalamus: a useful tool in stereotactic surgery." STEREOTACTIC AND FUNCTIONAL NEUROSURGERY, (1993) 60 (4) 168-74. , XP002117001

## Beschreibung

Die. Erfindung betrifft die Verwendung eines die Dopamin-Konzentration im synaptischen Spalt der Nervenzellen des Gehirns erhöhende Substanz enthaltenden Mittels zur Herstellung eines Medikaments zur Behandlung der Symptome der Parkinsonschen Krankheit.

In der klassischen Therapie der Parkinsonschen Kankheit wird als Hauptwirkstoff LevoDopa, auch als L-Dopa bekannt, eingesetzt. LevoDopa ist eine Vorstufe von Dopamin und im Gegensatz zu letzterem in der Lage, nach einer Applikation die Blut-Hirn-Schranke zu passieren. Nach einem Passieren der Blut-Hirn-Schranke wird LevoDopa im Gehirn zu Dopamin umgewandelt. Die Substanz Dopamin wirkt im Gehirn als Neurotransmitter im synaptischen Spalt der Nervenzellen des Gehirns in der Weise, dass eine Signalübertragung von einer Zelle zu einer anderen gefördert wird. Die Konzentration von Dopamin ist im Gehirn von an der Parkinsonschen Krankheit leidenden Personen in zu niedriger Konzentration vorhanden, so dass eine Signalübertragung von einer Nervenzelle zu einer anderen im Gehirn beeinträchtigt ist. Durch die Gabe von LevoDopa bei Parkinson-Patienten wird die Dopamin-Konzentration im synaptischen Spalt der Nervenzellen des Gehirns erhöht, so dass die Signalübertragung zwischen den Nervenzellen des Gehirns verbessert wird und eine verbesserte Kontrolle über motorische und intellektuelle Vorgänge herbeigeführt wird.

Zusätzlich zu LevoDopa werden in der klassischen Therapie der Parkinsonschen Krankheit Patienten Substanzen verabreicht, die die Wirkung von Dopamin fördern und als Dopamin-fördernde Agonisten bezeichnet sind. Die Dopamin-fördernden Agonisten wirken dabei generell in der Weise, dass die Dopamin-Konzentration im synaptischen Spalt der Nervenzellen des Gehirns durch Hemmung des dortigen Abbaus von Dopamin in erhöhter Konzentration vorhanden bleibt. Der normale Abbau von Dopamin wird dadurch verursacht, dass es durch ein Enzym, der Monooxygenase, in Noradrenalin abgebaut wird. Die Bildung von Monooxygenase kann durch bestimmte Dopamin-fördernde Agonisten gehemmt werden, so dass Dopamin nur in gemindertem Maße in Noradrenalin abgebaut werden kann und eine gegebene Konzentration von Dopamin im synaptischen Spalt somit länger erhalten bleibt. Alternativ kann die Wirkungsweise von Dopamin-fördernden Agonisten darauf beruhen, dass in Speichern des Gehirns gespeichertes Dopamin freigesetzt wird und die Wiederaufnahme in den Speicher gehemmt wird. Zu den Dopamin-fördernden Agonisten zählen Bromocriptin, Selegilin, Amantadin, Pergolid-Mesilat oder Tolcapon. Als weiterer Dopamin-fördernder Agonist ist Normabrain (Piracetam) bekannt, dessen Wirkung darauf beruht, dass die Durchblutung des Gehirns allgemein verbessert wird.

Aufgabe der Erfindung ist es, ein Medikament zu finden, das für sich allein, insbesondere jedoch in Kombination mit den bekannten Substanzen, die Symptome der Parkinsonschen Krankheit mindert.

Erfindungsgemäß wird diese Aufgabe dadurch gelöst, dass zur Herstellung des Medikamentes ein Mittel verwendet wird, das
eine die Dopamin-Konzentration im synaptischen Spalt der Nervenzellen des Gehirns erhöhende Substanz und ein Lokalanästhetikum der Anilid-Gruppe oder dessen Derivate enthält.

Bevorzugte Ausführungsformen der Erfindung sind Gegenstand der Unteransprüche.

Gemäß einer bevorzugten Ausführungsform der erfindungsgemäßen Verwendung ist als Lokalanästhetikum der Anilid-Gruppe die Substanz Mepivacain gewählt, vorzugsweise in einer Tagesdosis von 30 mg bis 60 mg. Alternativ zu Mepivacain können die Substanzen Lidocain, Bupivacain, Butanilicain, Tolycain oder Etidocain verwendet werden.

Bei Applikation von Lidocain oder Bupivacain wird eine Tagesdosis bis zu 150mg empfohlen.

Die erfindungsgemäße Verwendung hat bei Applikation des Medikaments bei Parkinson-Patienten die Wirkung, dass die spezifischen Symptome der Parkinson-Krankheit deutlich zurückgehen, wobei der so verursachte verbesserte Zustand der Patienten über mehrere Stunden und auch Tage anhält. Insbesondere wurde bei Applikation des durch die erfindungsgemäße Verwendung hergestellten Medikaments eine deutliche Verbesserung dahin gehend erzielt, dass:
- die Motorik und Feinmotorik verbessert
- die Beweglichkeit erhöht
- die Konzentrationsfähigkeit erhöht
- die Reaktionszeit vermindert
- die Aussprache verbessert
- das Auffassungsvermögen verbessert
- die Psyche erhellt und Stimmungslage verbessert wurde.

Bei der erfindungsgemäßen Verwendung wird durch die Kombination einer die Dopamin-Konzentration im synaptischen Spalt der Nervenzellen des Gehirns erhöhenden Substanz mit einem Lokalanästhetikum der Anilid-Gruppe möglicherweise erreicht, dass die Permeabilität der Blut-Hirn-Schranke für die Substanz LevoDopa erhöht wird, so dass Dopamin in höherer Konzentration als bei einer Standardtherapie im Gehirn von an der Parkinsonschen Krankheit leidenden Personen anreicherbar ist, wodurch folglich eine höhere Konzentration von Dopamin im Gehirn dieser Personen erreicht wird. Darüber hinaus wird die Verweildauer von Dopamin im Gehirn möglicherweise erhöht.

Die Substanz "Lokalanästhetikum der Anilid-Gruppe" gehört generell zu den Lokalanästhetika unterschiedlicher Struktur, wobei als Untergruppe dieser Lokalanästhetika die Lokalanästhetika der Anilid-Gruppe und deren Derivate zur Therapie bevorzugt werden. Ausführungsbeispiele dieser Untergruppe sind neben Mepivacain, Lidocain, Bupivacain, Butanilicain, Etidocain und Tolycain. Das kleinste Molekül dieser genannten Gruppe hat Mepivacain, und diese Substanz hat sich auch am wirkungsvollsten bei der Therapie von Patienten der Parkinsonschen Krankheit erwiesen. Eine Vermutung dabei ist, dass aufgrund der geringen Molekülgröße des Mepivacain eine erhöhte Wahrscheinlichkeit zum Durchgang der Blut-Hirn-Schranke gegeben ist. Mepivacain ist darüber hinaus lipophil, d.h. fettliebend und setzt sich gerne an Fettmoleküle an. In diesem Zusammenhang ist es bemerkenswert; dass Nervenzellen meist in Fett eingebettet sind und eine Anlage oder Anreicherung von Mepivacain in Fett auch Auswirkungen auf die durch das Fettgewebe verlaufenden Nervenbahnen zeitigen dürfte. Auch LevoDopa besitzt ähnlich wie Mepivacain eine starke Lipophilie, so dass auch über diesen Zusammenhang ein möglicher Wirkmechanismus gegeben ist.

Bei einer Ausführungsform der erfindungsgemäßen Verwendung enthält das Mittel zur Herstellung eines Medikaments zur Behandlung der Parkinsonschen Krankheit LevoDopa, das vorzugsweise in einer Tagesdosis von 200 mg bis 600 mg appliziert wird.

Gemäß einer alternativen Ausführungsform der erfindungsgemäßen Verwendung enthält die die Dopamin-Konzentration im synaptischen Spalt der Nervenzellen des Gehirns erhöhende Substanz zusätzlich Bromocriptin, das vorzugsweise in einer Tagesdosis von 1,0 oder 1,25 mg bis 10 mg appliziert wird.

Gemäß einer weiteren Ausführungsform der erfindungsgemäßen Verwendung enthält die die Dopamin-Konzentration im synaptischen Spalt der Nervenzellen des Gehirns erhöhende Substanz zusätzlich Selegilin, das vorzugsweise in einer Tagesdosis von 4 mg bis 20 mg appliziert wird.

Gemäß einer weiteren alternativen Ausführungsform der erfindungsgemäßen Verwendung enthält die die Dopamin-Konzentration im synaptischen Spalt der Nervenzellen des Gehirns erhöhende Substanz zusätzlich Amantadin, das vorzugsweise in einer Tagesdosis von 100 mg bis 400 mg appliziert wird.

Gemäß einer anderen alternativen Ausführungsform der erfindungsgemäßen Verwendung enthält die die Dopamin-Konzentration im synaptischen Spalt der Nervenzellen des Gehirns erhöhende Substanz zusätzlich Pergolid-Mesilat, das vorzugsweise in einer Tagesdosis von 2 mg bis 8 mg appliziert wird.

Bei einer anderen Ausführungsform der Erfindung kann das verwendete Mittel als eine die Dopamin-Konzentration im synaptischen Spalt der Nervenzellen des Gehirns erhöhende Substanz auch Tolcapon enthalten, das in einer Tagesdosis von 100 mg bis 400 mg appliziert wird.

Gemäß einer weiteren erfindungsgemäßen Ausführungsform der erfindungsgemäßen Verwendung könnte die die Dopamin-Konzentration im synaptischen Spalt der Nervenzellen des Gehirns erhöhende Substanz zusätzlich Piracetam enthalten, das in einer Tagesdosis von 1.000 mg bis 4.000 mg appliziert wird.

Die oben angegebenen Substanzen, die die Dopamin-Konzentration im synaptischen Spalt der Nervenzellen des Gehirns erhöhen, können bei der erfindungsgemäßen Verwendung jeweils für sich als auch in unterschiedlichen Kombinationen miteinander in dem erfindungsgemäß verwendeten Mittel enthalten sein. Die Wirkung des erfindungsgemäß hergestellten Medikaments beruht jedoch weniger auf einer speziellen Kombination von die Dopamin-Konzentration im synaptischen Spalt der Nervenzellen des Gehirns erhöhenden Substanzen der klassischen Parkinson-Therapie untereinander, als vielmehr auf einer Kombination dieser klassisch zur Parkinson-Therapie eingesetzten Substanzen mit einem Lokalanästhetikum der Anilid-Gruppe und hierbei insbesondere, jedoch nicht ausschließlich, mit der Substanz Mepivacain.

Die angegebenen Dosierungen der Lokalanästhetika sind auf Injektions-Applikationen bezogen. Bei oraler Applikation ist die Dosierung entsprechend anzupassen.

## Patentansprüche

1. Verwendung eines Mittels enthaltend eine die Dopamin-Konzentration im synaptischen Spalt der Nervenzellen des Gehirns erhöhende Substanz und ein Lokalanästhetikum der Anilid-Gruppe oder dessen Derivate zur Herstellung eines Medikaments zur Behandlung der Symptome der Parkinsonschen Krankheit.

2. Verwendung nach Anspruch 1, **dadurch gekennzeichnet, dass** als Lokalanästhetikum der Anilid-Gruppe oder dessen Derivat der Wirkstoff Mepivacain vorgesehen ist.

3. Verwendung nach Anspruch 2, **dadurch gekennzeichnet, dass** das Mepivacain in einer Tagesdosis von 30 mg bis 60 mg zu applizieren ist.

4. Verwendung nach Anspruch 1, **dadurch gekennzeichnet, dass** als Lokalanästhetikum der Anilid-Gruppe oder dessen Derivat der Wirkstoff Lidocain vorgesehen ist.

5. Verwendung nach Anspruch 4, **dadurch gekennzeichnet, dass** das Lidocain in einer Tagesdosis von bis zu 150 mg zu applizieren ist.

6. Verwendung nach Anspruch 1, **dadurch gekennzeichnet, dass** als Lokalanästhetikum der Anilid-Gruppe oder dessen Derivat der Wirkstoff Bupivacain vorgesehen ist.

7. Verwendung nach Anspruch 6, **dadurch gekennzeichnet, dass** das Bupivacain in einer Tagesdosis von bis zu 150 mg zu applizieren ist.

8. Verwendung nach Anspruch 1, **dadurch gekennzeichnet, dass** als Lokalanästhetikum der Anilid-Gruppe oder dessen Derivat der Wirkstoff Butanilicain vorgesehen ist.

9. Verwendung nach Anspruch 1, **dadurch gekennzeichnet, dass** als Lokalanästhetikum der Anilid-Gruppe oder dessen Derivat der Wirkstoff Tolycain vorgesehen ist.

10. Verwendung nach Anspruch 1, **dadurch gekennzeichnet, dass** als Lokalanästhetikum der Anilid-Gruppe oder dessen Derivat der Wirkstoff Etidocain vorgesehen ist.

11. Verwendung zur Herstellung eines Medikaments zur Behandlung der Symptome der Parkinsonschen Krankheit nach einem der vorgehergehenden Ansprüche, **dadurch gekennzeichnet, dass** die die Dopamin-Konzentration im synaptischen Spalt der Nervenzellen des Gehirns erhöhende Substanz LevoDopa enthält.

12. Verwendung nach Anspruch 11, **dadurch gekennzeichnet, dass** das LevoDopa in einer Tagesdosis von 200 mg bis 600 mg zu applizieren ist.

13. Verwendung nach einem der Ansprüche 11 oder 12, **dadurch gekennzeichnet, dass** die die Dopamin-Konzentration im synaptischen Spalt der Nervenzellen des Gehirns erhöhende Substanz zusätzlich Bromocriptin enthält.

14. Verwendung nach Anspruch 13, **dadurch gekennzeichnet, dass** das Bromocriptin in einer Tagesdosis von 1,0 mg bis 10 mg zu applizieren ist.

15. Verwendung nach einem der Ansprüche 11 bis 14, **dadurch gekennzeichnet, dass** die die Dopamin-Konzentration im synaptischen Spalt der Nervenzellen des Gehirns erhöhende Substanz zusätzlich Selegilin enthält.

16. Verwendung nach Anspruch 15, **dadurch gekennzeichnet, dass** das Selegilin in einer Tagesdosis von 4 mg bis 20 mg zu applizieren ist.

17. Verwendung nach einem der Ansprüche 11 bis 16, **dadurch gekennzeichnet, dass** die die Dopamin-Konzentration im synaptischen Spalt der Nervenzellen des Gehirns erhöhende Substanz zusätzlich Amantadin enthält.

18. Verwendung nach Anspruch 17, **dadurch gekennzeichnet, dass** das Amantadin in einer Tagesdosis von 100 mg bis 400 mg zu applizieren ist.

19. Verwendung nach einem der Ansprüche 11 bis 18, **dadurch gekennzeichnet, dass** die die Dopamin-Konzentration im synaptischen Spalt der Nervenzellen des Gehirns erhöhende Substanz zusätzlich Pergolid-Mesilat enthält.

20. Verwendung nach Anspruch 19, **dadurch gekennzeichnet, dass** das Pergolid-Mesilat in einer Tagesdosis von 2 mg bis 8 mg zu applizieren ist.

21. Verwendung nach einem der Ansprüche 11 bis 20, dadurch gekennzeichent, dass die die Dopamin-Konzentration im synaptischen Spalt der Nervenzellen des Gehirns erhöhende Substanz zusätzlich Tolcapon enthält.

22. Verwendung nach Anspruch 21, **dadurch gekennzeichnet, dass** das Tolcapon in einer Tagesdosis von 100 mg bis 400 mg zu applizieren ist.

23. Verwendung nach einem der Ansprüche 11 bis 22, **dadurch gekennzeichnet, dass** die die Dopamin-Konzentration im synaptischen Spalt der Nervenzellen des Gehirns erhöhende Substanz zusätzlich Piracetam enthält.

24. Verwendung nach Anspruch 23, **dadurch gekennzeichnet, dass** das Piracetam in einer Tagesdosis von 1000 mg bis 4000 mg zu applizieren ist.

## Claims

1. Use of an agent having a substance increasing the dopamine concentration in the synaptic cleft of the neurons of the brain and a local anaesthetic of the anilide group or its derivatives for producing a medication for treating the symptoms of Parkinson's disease.

2. Use according to claim 1, **characterized in that** the effective substance mepivacaine is provided as local anaesthetic of the anilide group or its derivative.

3. Use according to claim 2, **characterized in that** mepivacaine is to be applied in a daily dose of 30 mg to 60 mg.

4. Use according to claim 1, **characterized in that** the effective substance lidocaine is provided as local anaesthetic of the anilide group or its derivative.

5. Use according to claim 4, **characterized in that** lidocaine is to be applied in a daily dose of up to 150 mg.

6. Use according to claim 1, **characterized in that** the effective substance bupivacaine is provided as local anaesthetic of the anilide group or its derivative.

7. Use according to claim 6, **characterized in that** bupivacaine is to be applied in a daily dose of up to 150 mg.

8. Use according to claim 1, **characterized in that** the effective substance butanilicaine Is provided as local anaesthetic of the anilide group or its derivative.

9. Use according to claim 1, **characterized in that** the effective substance tolycaine is provided as local anaesthetic of the anilide group or its derivative.

10. Use according to claim 1, **characterized In that** the effective substance etidocaine is provided as local anaesthetic of the anilide group or its derivative.

11. Use for producing a medication for treating the symptoms of Parkinson's disease according to any of the above claims, **characterized in that** the substance increasing the dopamine concentration in the synaptic cleft of the neurons of the brain contains levodopa.

12. Use according to claim 11, **characterized in that** levodopa is to be applied in a daily dose of 200 mg to 600 mg.

13. Use according to one of the claims 11 or 12, **characterized in that** the substance increasing the dopamine concentration in the synaptic cleft of the neurons of the brain additionally contains bromocriptine.

14. Use according to claim 13, **characterized in that** bromocriptine is to be applied in a daily dose of 1.0 mg to 10 mg.

15. Use according to any of claims 11 to 14, **characterized in that** the substance increasing the dopamine concentration in the synaptic cleft of the neurons of the brain additionally contains selegiline.

16. Use according to claim 15, **characterized in that** selegiline is to be applied in a daily dose of 4 mg to 20 mg.

17. Use according to any one of the claims 11 through 16, **characterized in that** the substance increasing the dopamine concentration in the synaptic cleft of the neurons of the brain additionally contains amantadine.

18. Use according to claim 17, **characterized in that** amantadine is to be applied in a daily dose of 100 mg to 400 mg.

19. Use according to any of claims 11 to 18, **characterized in that** the substance increasing the dopamine concentration in the synaptic cleft of the neurons of the brain additionally contains pergolide mesylate.

20. Use according to claim 19, **characterized in that** pergolide mesylate is to be applied in a daily dose of 2 mg to 8 mg.

21. Use according to any of claims 11 to 20, **characterized in that** the substance increasing the dopamine concentration in the synaptic cleft of the neurons of the brain additionally contains tolcapone.

22. Use according to claim 21, **characterized in that** tolcapone is to be applied in a daily dose of 100 mg to 400 mg.

23. Use according to any of claims 11 to 22, **characterized in that** the substance increasing the dopamine concentration in the synaptic cleft of the neurons of the brain additionally contains piracetam.

24. Use according to claim 23, **characterized in that** piracetam is to be applied in a daily dose of 1000 mg to 4000 mg.

## Revendications

1. Utilisation d'un agent contenant une substance augmentant la concentration de dopamine dans la fente synaptique des cellules nerveuses du cerveau et un anesthésique local du groupe des anilides ou ses dérivés pour la préparation d'un médicament pour le traitement des symptômes de la maladie de Parkinson.

2. Utilisation selon la revendication 1, **caractérisée en ce que** le principe actif mépivacaïne est prévu comme anesthésique local du groupe des anilides ou son dérivé.

3. Utilisation selon la revendication 2, **caractérisée en. ce que** la mépivacaïne doit être appliquée en une dose quotidienne de 30 mg à 60 mg.

4. Utilisation selon la revendication 1, **caractérisée en ce que** le principe actif lidocaïne est prévu comme anesthésique local du groupe des anilides ou son dérivé.

5. Utilisation selon la revendication 4, **caractérisée en ce que** la lidocaïne doit être appliquée en une dose quotidienne pouvant atteindre 150 mg.

6. Utilisation selon la revendication 1, **caractérisée en ce que** le principe actif bupivacaïne est prévu comme anesthésique local du groupe des anilides ou son dérivé.

7. Utilisation selon la revendication 6, caractérisée en ce la bupivacaïne doit être appliquée en une dose quotidienne pouvant atteindre 150 mg.

8. Utilisation selon la revendication 1, **caractérisée en ce que** le principe actif butanilicaïne est prévu comme anesthésique local du groupe des anilides ou son dérivé.

9. Utilisation selon la revendication 1, **caractérisée en ce que** le principe actif tolycaïne est prévu comme anesthésique local du groupe des anilides ou son dérivé.

10. Utilisation selon la revendication 1, **caractérisée en ce que** le principe actif étidocaïne est prévu comme anesthésique local du groupe des anilides ou son dérivé.

11. Utilisation pour la préparation d'un médicament pour le traitement des symptômes de la maladie de Parkinson selon l'une des revendications précédentes, **caractérisée en ce que** la substance augmentant la concentration de dopamine dans la fente synaptique des cellules nervures du cerveau contient de la lévodopa.

12. Utilisation selon la revendication 11, **caractérisée en ce que** la lévodopa doit être appliquée en une dose quotidienne de 200 mg à 600 mg.

13. Utilisation selon l'une des revendications 11 et 12, **caractérisée en ce que** la substance augmentant la concentration de dopamine dans la fente synaptique des cellules nerveuses du cerveau contient en outre de la bromocriptine.

14. Utilisation selon la revendication 13, **caractérisée en ce que** la bromocriptine doit être appliquée en une dose quotidienne de 1,0 mg à 10 mg.

15. Utilisation selon l'une des revendications 11 à 14, **caractérisée en ce que** la substance augmentant la concentration de dopamine dans la fente synaptique des cellules nerveuses du cerveau contient en outre de la sélégiline.

16. Utilisation selon la revendication 15, **caractérisée en ce que** la sélégiline doit être appliquée en une dose quotidienne de 4 mg à 20 mg.

17. Utilisation selon l'une des revendications 11 à 16, **caractérisée en ce que** la substance augmentant la concentration de dopamine dans la fente synaptique des cellules nerveuses du cerveau contient en outre de l'amantadine.

18. Utilisation selon la revendication 17, **caractérisée en ce que** l'amantadine doit être appliquée en une dose quotidienne de 100 mg à 400 mg.

19. Utilisation selon l'une des revendications 11 à 18, **caractérisée en ce que** la substance augmentant la concentration de dopamine dans la fente synaptique des cellules nerveuses du cerveau contient en outre du mésilate de pergolide.

20. Utilisation selon la revendication 19, **caractérisée en ce que** le mésilate de pergolide doit être appliqué en une dose quotidienne de 2 mg à 8 mg.

21. Utilisation selon l'une des revendications 11 à 20, **caractérisée en ce que** la substance augmentant la concentration de dopamine dans la fente synaptique des cellules nervures du cerveau contient en outre de la tolcapone.

22. Utilisation selon la revendication 21, **caractérisée en ce que** la tolcapone doit être appliquée en une dose quotidienne de 100 mg à 400 mg.

23. Utilisation selon l'une des revendications 11 à 22, **caractérisée en ce que** la substance augmentant la concentration de dopamine dans la fente synaptique des cellules nerveuses du cerveau contient en outre du piracétam.

24. Utilisation selon la revendication 23, **caractérisée en ce que** le piracétam doit être appliqué en une dose quotidienne de 1000 mg à 4000 mg.
